# EUROPEAN PATENT APPLICATION

(11) **EP 4 474 410 A1**
(43) Date of publication of application: **11.12.2024**
(21) Application number: 24177623.6
(22) Date of filing: 23.05.2024
(51) Int. Cl.: C08J 7/04, C12N 5/00, G01N 33/49

(54) **METHOD OF PRODUCING POLYMER-COATED SUBSTRATE FOR CAPTURING CANCER CELLS**

(30) Priority: 09.06.2023 JP 2023095828; 26.02.2024 JP 2024026388
(71) Applicant: Sumitomo Rubber Industries, Ltd., Kobe-shi, Hyogo 651-0072 (JP); Kyushu University, National University Corporation, Nishi-ku Fukuoka-shi Fukuoka 8190395 (JP)
(72) Inventor: MINAGAWA, Yasuhisa, Kobe-shi, 6510072 (JP); TANAKA, Masaru, Fukuoka-shi, 8190395 (JP); ANADA, Takahisa, Fukuoka-shi, 8190395 (JP); YAMAMOTO, Aki, Fukuoka-shi, 8190395 (JP); SHINDO, Hironori, Kishiwada-shi, 596-0049 (JP); PARK, Jisu, Kishiwada-shi, 596-0049 (JP); UJIGAWA, Kazuma, Kishiwada-shi, 596-0049 (JP)
(74) Representative: Manitz Finsterwald Patent- und Rechtsanwaltspartnerschaft mbB

(57) **Abstract**

Provided is a method of producing a polymer-coated substrate for capturing cancer cells in which a polymer layer is stably maintained on the surface of the substrate for a long time even in a liquid environment. The method of producing a polymer-coated substrate for capturing cancer cells includes forming a polymer layer using a hydrophilic polymer represented by the following formula (I) on a surface of a substrate having undergone antireflection treatment, 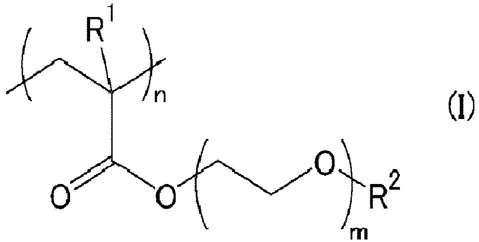 wherein R¹ represents a hydrogen atom or a methyl group; R² represents an alkyl group; m represents 1 to 5; and n represents the number of repetitions.

## Description

### TECHNICAL FIELD

The present invention relates to a method of producing a polymer-coated substrate for capturing cancer cells.

### BACKGROUND ART

Coating the surfaces of substrates with special polymers has been proposed to produce devices for capturing specific cells (blood cells, cancer cells, etc. present in blood or biological fluid) from blood or biological fluid.

However, in the case of a glass substrate, for example, some special polymer materials do not have high affinity with glass. When a polymer material not having high affinity is coated on a substrate to form a polymer film, the coating film may be peeled off the substrate and may partially lift up or may be separated in a liquid environment such as in water. The lifting or separation affects the capturing ability. For example, the coating film may separate after it captures cancer cells. There is a demand for polymer-coated substrates for capturing cancer cells in which the polymer layer is stably formed (applied) on the surface of the substrate for a long time even in a liquid environment.

### SUMMARY OF INVENTION

### TECHNICAL PROBLEM

The present invention aims to solve the above problem and to provide a method of producing a polymer-coated substrate for capturing cancer cells in which a polymer layer is stably maintained on the surface of the substrate for a long time even in a liquid environment.

### SOLUTION TO PROBLEM

The present invention relates to a method of producing a polymer-coated substrate for capturing cancer cells, the method including forming a polymer layer using a hydrophilic polymer represented by the following formula (I) on a surface of a substrate having undergone anti-reflection treatment, 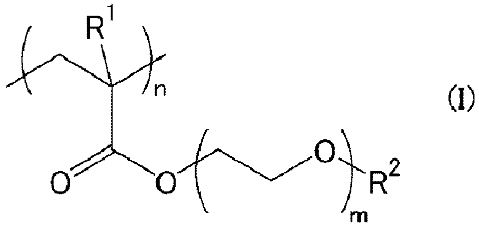 wherein R¹ represents a hydrogen atom or a methyl group; R² represents an alkyl group; m represents 1 to 5; and n represents the number of repetitions.

### ADVANTAGEOUS EFFECTS OF INVENTION

The method of producing a polymer-coated substrate for capturing cancer cells according to the present invention includes forming a polymer layer using a hydrophilic polymer represented by the formula (I) on a surface of a substrate having undergone anti-reflection treatment. The polymer layer is strongly adsorbed to and coats the surface of the substrate even in a liquid environment such as in water, so that the polymer layer is prevented from lifting or separating. Thus, a polymer-coated substrate for capturing cancer cells in which the polymer layer is stably maintained on the surface of the substrate for a long time can be provided. Cancer cell-capturing ability can be improved by using a polymer-coated substrate for capturing cancer cells produced by the method.

### DESCRIPTION OF EMBODIMENTS

The present invention relates to a method of producing a polymer-coated substrate for capturing cancer cells including forming a polymer layer using a hydrophilic polymer represented by the formula (I) on a surface of a substrate having undergone anti-reflection treatment.

The number of tumor cells (cancer cells, etc.) appearing in biological fluid, such as circulating tumor cells (several to hundreds of cells/1 ml of blood), is very small, and it is considered important to capture tumor cells present in the sampled biological fluid as many as possible to analyze them. The production method of the present invention includes forming a polymer layer using a hydrophilic polymer represented by the formula (I) on a surface of a substrate having undergone anti-reflection treatment. The method enables production of a polymer-coated substrate for capturing cancer cells in which the polymer is firmly adsorbed to the surface of the substrate to be stably maintained for a long time without lifting or separation. Instability such as lifting or separation of the polymer layer affects the capture of cancer cells. Excellent cancer cell-capturing ability can be achieved by improving the stability (long-term stable coating properties). The number of cancer cells in biological fluid can be determined by measuring the number of cancer cells captured with the polymer layer of a produced polymer-coated substrate for capturing cancer cells, and thus the cancer-treating effect, etc. can be confirmed. Moreover, the captured cancer cells may be cultured and then used to determine the effects of drugs such as anticancer drugs. Thus, the effects of drugs such as anticancer drugs ex vivo can be confirmed before administration. This technique is also useful to screen drugs such as anticancer drugs. Further, genetic analysis of the captured cancer cells is useful to screen drugs such as anticancer drugs.

(Method of producing polymer-coated substrate for capturing cancer cells)

The method of producing a polymer-coated substrate for capturing cancer cells according to the present invention includes forming a polymer layer using a hydrophilic polymer represented by the formula (I) on a surface of a substrate having undergone anti-reflection treatment.

Herein, the term "anti-reflection treatment" refers to a treatment to suppress surface reflection.

Examples of the treatment to suppress surface reflection include anti-reflection treatments (non-glare treatment) performed by forming irregularities on the surface of the substrate by spraying fine glass particles or by applying a special coating to the surface.

Specific examples of the anti-reflection treatment include non-reflective treatment, low reflection treatment, polarizing treatment, and non-glare treatment. These may be performed alone or in combinations of two or more.

The substrate having undergone anti-reflection treatment has a 60-degree gloss (glossiness at 60 degree) of preferably 150% or less, more preferably 1300 or less but preferably 50% or more, more preferably 700 or more.

Herein, the term "60-degree gloss" is a value determined by measuring the 60-degree gloss of a center portion of the substrate having undergone anti-reflection treatment using a gloss meter (IG320, HORIBA) in accordance with JIS-K 7150.

Non-limiting examples of the substrate include glass, acrylic resin, methacrylic resin, polyester resin, cycloolefin resin, and silicon resin. Glass substrates are preferred among these.

The type of the glass in the glass substrates is not limited. Examples include soda lime glass, alkali-free glass, borosilicate glass (SiO₂-B₂O₃-ZnO glass, SiO₂-B₂O₃-Bi₂O₃ glass, etc.), potash glass, crystal glass (glass containing PbO, such as SiO₂-PbO glass, SiO₂-PbO-B₂O₃ glass, SiO₂-B₂O₃-PbO glass, etc.), titanium crystal glass, barium glass, boron glass (B₂O₃-ZnO-PbO glass, B₂O₃-ZnO-Bi₂O₃ glass, B₂O₃-Bi₂O₃ glass, B₂O₃-ZnO glass, etc.), strontium glass, alumina silicate glass, soda-zinc glass, and soda-barium glass (BaO-SiO₂ glass, etc.). The glass may be used alone or in combinations of two or more.

Although the thickness of the substrate having undergone anti-reflection treatment is not limited, the average thickness is preferably 100 um or more and 5000 um or less, more preferably 100 um or more and 3000 um or less. The term "average thickness" refers to an average of thicknesses measured at ten arbitrary points using a micrometer.

The polymer layer is formed using a hydrophilic polymer (polymer with hydrophilicity) represented by the following formula (I): 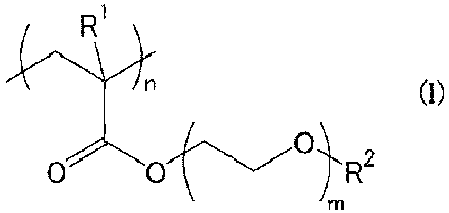 wherein R¹ represents a hydrogen atom or a methyl group; R² represents an alkyl group; m represents 1 to 5; and n represents the number of repetitions. The hydrophilic polymer represented by the formula (I) may be used alone or in combinations of two or more.

The carbon number of the alkyl group for R² is preferably 1 to 10, more preferably 1 to 5. R² is particularly preferably a methyl group or an ethyl group. The symbol m is preferably 1 to 3. The symbol n (the number of repeating units) is preferably 70 to 2500, more preferably 90 to 1500.

Examples of hydrophilic polymers represented by the formula (I) include homopolymers or copolymers of one or two or more hydrophilic monomers. The hydrophilic polymer may be used alone or in combinations of two or more.

Examples of the homopolymers or copolymers of the hydrophilic monomers include polyalkoxyalkyl methacrylates such as polymethoxyethyl(meth)acrylate. Polyalkoxyalkyl acrylate is preferred, and poly(2-methoxyethyl acrylate) is more preferred among these.

Specific examples of the hydrophilic monomer constituting the hydrophilic polymers represented by the formula (I) include alkoxyalkyl(meth)acrylates such as methoxyethyl(meth)acrylate. Alkoxyalkyl acrylate is preferred, and 2-methoxyethyl acrylate is more preferred among these. The hydrophilic monomer may be used alone or in combinations of two or more.

The step of forming a polymer layer using a hydrophilic polymer represented by the formula (I) on the surface of the substrate having undergone anti-reflection treatment may include forming two or more layers of the polymer layer. The two or more polymer layers may be formed using the same hydrophilic polymer or different hydrophilic polymers. To better achieve the advantageous effect, the two or more polymer layers are desirably formed using the same hydrophilic polymer.

The hydrophilic polymer(s) represented by the formula (I) constituting the two or more polymer layers may be appropriately selected. The hydrophilic polymer represented by the formula (I) may be used alone or in combinations of two or more in each polymer layer.

The Mn of the hydrophilic polymer constituting the outermost layer (a polymer layer on the outermost side (surface side)) among the two or more polymer layers is preferably 5000 or more, more preferably 6000 or more, still more preferably 7000 or more, while it is preferably 30000 or less, more preferably 25000 or less, still more preferably 20000 or less.

To better achieve the advantageous effect, the number average molecular weight of the hydrophilic polymer constituting a polymer layer on the surface of the substrate having undergone anti-reflection treatment (a polymer layer in contact with the substrate having undergone anti-reflection treatment (hereinafter, also referred to as innermost layer) among the two or more polymer layers is preferably larger than the number average molecular weight of the hydrophilic polymer constituting any other polymer layer (one or more polymer layers other than the innermost layer among the two or more polymer layers).

The Mn of the hydrophilic polymer constituting any other polymer layer among the two or more polymer layers is preferably 5000 or more, more preferably 6000 or more, still more preferably 7000 or more, while it is preferably 30000 or less, more preferably 25000 or less, still more preferably 20000 or less.

When the number average molecular weight of the hydrophilic polymer constituting the innermost layer among the two or more polymer layers is larger than the number average molecular weight of the hydrophilic polymer constituting the polymer layer other than the innermost layer, the Mn of the hydrophilic polymer constituting the innermost layer is preferably 32000 or more, more preferably 40000 or more, still more preferably 60000 or more, while it is preferably 200000 or less, more preferably 150000 or less, still more preferably 120000 or less. When the Mn is within the range indicated above, the advantageous effect tends to be better achieved.

Herein, the number average molecular weight (Mn) can be determined with a gel permeation chromatograph (GPC) (GPC-8000 series available from Tosoh Corporation, detector: differential refractometer, column: TSKgel SuperMultipore HZ-M available from Tosoh Corporation). The resulting value is calibrated with polystyrene standards.

The total thickness (total film thickness) of one layer or two or more layers of the polymer layer (one hydrophilic polymer layer or two or more hydrophilic polymer layers formed using a hydrophilic polymer represented by the formula (I)) is preferably 10 to 1000 nm, more preferably 30 to 700 nm, still more preferably 50 to 350 nm. When the thickness is controlled to be within the range indicated above, low adsorption of normal proteins and cells and selective capture of cancer cells can be expected.

The surface of the polymer layer (the surface of the polymer layer of the polymer-coated substrate for capturing cancer cells) preferably at least partially (partially or entirely) has a water contact angle of not larger than 60 degrees, more preferably not larger than 50 degrees. The lower limit is not limited, and a smaller angle is better.

The polymer-coated substrate for capturing cancer cells in which the polymer layer is formed entirely or partly on the surface of the substrate having undergone anti-reflection treatment can be produced by coating (covering) entirely or partly the surface of the substrate having undergone anti-reflection treatment with polymers using known techniques. Examples of the techniques include a method 1) of introducing a polymer solution or dispersion in which a hydrophilic polymer represented by the formula (I) is dissolved or dispersed in a solvent onto the surface (recess(es) of the substrate) of the substrate having undergone anti-reflection treatment, followed by retention for a predetermined time and optional drying, and a method 2) of applying (spraying) the polymer solution or dispersion to the surface of the substrate having undergone anti-reflection treatment, optionally followed by drying for a predetermined time. Then, the polymer-coated substrate for capturing cancer cells may be combined with other components as needed. Thereby, a device capable of, for example, capturing, culturing, and/or analyzing cancer cells can be produced.

Conventionally known materials or methods are applicable as the solvent, introduction method, application (spraying) method, etc.

The duration of retention/drying in the method 1) or 2) may be appropriately selected depending on the size of the substrate, the type of liquid introduced, etc. The duration of retention is preferably 10 seconds to 10 hours, more preferably 1 minute to 5 hours, still more preferably 5 minutes to 2 hours. The drying is preferably performed at room temperature (about 23°C) to 80°C, more preferably at room temperature to 60°C. The drying may be performed under reduced pressure. After the predetermined duration of retention, the excess polymer solution or dispersion may be discharged as appropriate before drying.

The solvent may be any solvent that can dissolve polymers and may be appropriately selected depending on the polymers used. The solvent may be water, an organic solvent, or a mixture thereof. Examples of the organic solvent include alcohols such as methanol, ethanol, n-propanol, i-propanol, and methoxypropanol; ketones such as acetone and methyl ethyl ketone; tetrahydrofuran, acetonitrile, ethyl acetate, and toluene.

The concentration of the polymer solution or dispersion is not limited and may be appropriately selected, taking the ease of introduction, applying, and spraying, productivity, etc. into consideration. The concentration of the polymers based on 100% by mass of the polymer solution or dispersion is preferably 0.1 to 10.0% by mass, more preferably 0.2 to 5.0% by mass.

When a polymer layer is formed using a hydrophilic polymer represented by the formula (I) on the surface of the substrate having undergone anti-reflection treatment according to the production method, the polymer layer is strongly adsorbed to and coats the surface of the glass substrate even in a liquid environment such as in water, so that the polymer layer is prevented from lifting or separating. Thus, a polymer-coated substrate for capturing cancer cells in which a polymer layer is stably maintained on the surface of the substrate for a long time can be provided.

Since the polymer-coated substrate for capturing cancer cells can capture cancer cells, the capturing effect can be exhibited when a sample (blood or biological fluid) is brought into contact with the polymer-coated substrate for capturing cancer cells in which a polymer layer is formed using a hydrophilic polymer represented by the formula (I) on the surface of the substrate having undergone anti-reflection treatment. The number of cancer cells in the sampled blood or biological fluid can be determined by measuring the number of the captured cancer cells, whereby the cancer-treating effect, etc. is expected to be confirmed.

### EXAMPLES

The preset invention is specifically described based on examples. However, the present invention is not limited to the examples.

### (Preparation of hydrophilic polymer)

Using a 12.5 mg/mL solution of azobisisobutyronitrile (AIBN) in toluene, 2-methoxyethyl acrylate (25 wt% in toluene) was thermally polymerized at 60°C for seven hours to prepare poly(2-methoxyethyl acrylate) (PMEA).

### (Examples 1 to 4)

The surface of a flat glass (made of soda lime glass, average thickness 1.35 mm) was subjected to non-glare treatment. Then, the flat glass was washed and dried with methanol, whereby a non-glare glass substrate shown in Table 1 was obtained.

A methanol solution of the PMEA at a concentration indicated in Table 1 was introduced into recesses of the obtained non-glare glass substrate, immediately followed by vacuum drying in an oven at 40°C for five minutes (coating). Thus, a polymer-coated glass substrate was prepared.

### (Comparative Example 1)

A methanol solution of the PMEA at a concentration indicated in Table 1 was introduced into recesses of a flat glass (made of soda lime glass, average thickness 1.35 mm) substrate, immediately followed by vacuum drying in an oven at 40°C for five minutes (coating). Thus, a polymer-coated glass substrate was prepared.

### (Examples 5 and 6, Comparative Example 2)

Polymer-coated glass substrates were produced as in Example 1, except that the Mn of the PMEA, the concentration and the fed amount of the methanol solution of the PMEA, and the non-glare treatment were changed according to Table 2.

A polymer 2 (Mn: 80000) as PMEA was hardly dissolved in methanol at room temperature. Thus, the polymer was warmed to 40°C for dissolution, whereby a polymer solution was prepared.

In Comparative Example 2 and Example 5, only one hydrophilic polymer layer was formed.

The polymer-coated glass substrates produced in the examples and the comparative examples were evaluated by the following methods.

[Analysis of blood spiked with cancer cells (Examples 1 to 4, Comparative Example 1, Table 1)]

Stained human colon adenocarcinoma (HT-29) cells were spiked in blood to prepare spiked blood (1000 HT29 cells/1 mL of blood). To the spiked blood was added 10% RosetteSep Human CD45 Depletion Cocktail, and the mixture was concentrated by centrifugation using Lypmhoprep. Then, the mononuclear cell layer was separated. PBS was added to the mononuclear cell layer, followed by centrifugation twice. After the centrifugation, the aggregates at the lowermost layer were suspended in a liquid medium containing 10% fetal bovine serum (FBS) in a liquid volume equal to the initial whole blood volume. An amount of 1.3 mL of the suspension was introduced into a chamber (polymer-coated glass substrate) and left at 37°C for 21 hours to cause adhesion. Thereafter, non-adhered cells were washed away with a PBS solution. Next, the numbers of adhered cancer cells (HT-29) and remaining leucocytes were counted with a fluorescence microscope. The number of cancer cells and the percentage (%) of "number of leucocytes/number of cancer cells × 100" were determined from the count numbers.

[Analysis of blood spiked with cancer cells (Examples 5 and 6, Comparative Example 2, Table 2)]

Stained human colon adenocarcinoma (HT-29) cells were spiked in blood to prepare spiked blood (500 HT29 cells/1 mL of blood). To the spiked blood was added 10% RosetteSep Human CD45 Depletion Cocktail, and the mixture was concentrated by centrifugation using Lypmhoprep. Then, the mononuclear cell layer was separated. PBS was added to the mononuclear cell layer, followed by centrifugation twice. After the centrifugation, the aggregates at the lowermost layer were suspended in a liquid medium containing 10% fetal bovine serum (FBS) in a liquid volume equal to the initial whole blood volume. An amount of 1.3 mL of the suspension was introduced into a chamber (polymer-coated glass substrate) and left at 37°C for 24 hours to cause adhesion. Thereafter, non-adhered cells were washed away with a PBS solution. Next, the number of adhered cancer cells (HT-29) was counted with a fluorescence microscope. The number of cancer cells (relative value) was determined from the count number.

### [Evaluation on separation of polymer layer in PBS]

The polymer-coated glass substrates produced as above were immersed in phosphate-buffered saline (PBS), and separation was observed after 1 hour, 24 hours, and 48 hours.

**[Table 1]**

| | | Comparative Example 1 | Example 1 | Example 2 | Example 3 | Example 4 |
|---|---|---|---|---|---|---|
| Concentration of PMEA methanol solution (% by mass) | | 0.5% | 0.5% | 0.5% | 0.5% | 0.5% |
| Fed amount of PMEA methanol solution | | 90 µl | 90 µl | 90 µl | 90 µl | 90 µl |
| 60-degree gloss (%) of surface of glass substrate | | Flat surface (outside the 150-50% range) | 130 | 110 | 90 | 70 |
| Number of cancer cells (relative value) | | 100 | 105 | 110 | 129 | 112 |
| Number of leucocytes/Number of cancer cells × 100 (%) | | 37 | 30 | 30 | 28 | 29 |
| Evaluation on separation of polymer layer in PBS | After one hour | Lifting at many parts | Not observed | Not observed | Not observed | Not observed |
| | After 24 hours | Lifting at many parts | Not observed | Not observed | Not observed | Not observed |
| | After 48 hours | Separation | Not observed | Not observed | Not observed | Not observed |

**[Table 2]**

| | | Comparative Example 2 | Example 5 | Example 6 |
|---|---|---|---|---|
| First layer (innermost layer) | Polymer species | PMEA | PMEA | PMEA |
| | Mn of PMEA | 16000 (Polymer 1) | 16000 (Polymer 1) | 80000 (Polymer 2) |
| | Concentration of PMEA methanol solution | 0.25% | 0.25% | 0.04% |
| | Fed amount of PMEA methanol solution | 90µl | 90µl | 80µl |
| Second layer (outermost layer) | Polymer species | - | - | PMEA |
| | Mn of PMEA | - | - | 16000 (Polymer 1) |
| | Concentration of PMEA methanol solution | - | - | 0.19% |
| | Fed amount of PMEA methanol solution | - | - | 90µl |
| 60-degree gloss (%) of surface of glass substrate | | Without non-glare treatment (outside the 150-50% range) | 90 | 90 |
| Number of cancer cells (relative value) | | 100 | 124 | 133 |
| Evaluation on separation of polymer layer in PBS | After one hour | Lifting at many parts | Not observed | Not observed |
| | After 24 hours | Lifting at many parts | Not observed | Not observed |
| | After 48 hours | Separation | Lifting at very small part | Not observed |

In Comparative Example 1, when immersed in PBS, the polymer layer increasingly lifted up over time and was finally separated.

In contrast, in Examples 1 to 4, lifting of the polymer layer was not observed even after 48 hours.

In the examples, the percentage (%) of "number of leucocytes/number of cancer cells × 100" was smaller than that in Comparative Example 1, and therefore cancer cells could be selectively captured. In Examples 1 to 4 using a substrate having a 60-degree gloss of 150% to 50%, selective capture was especially good.

In Examples 5 and 6 using a substrate having a 60-degree gloss of 90%, the number of adhered cancer cells (relative value) was larger than that in Comparative Example 2 using a substrate without non-glare treatment. The number was particularly large in Example 6 in which two polymer layers were formed.

Exemplary embodiments of the present invention include the following.

Embodiment 1. A method of producing a polymer-coated substrate for capturing cancer cells, the method including
forming a polymer layer using a hydrophilic polymer represented by the following formula (I) on a surface of a substrate having undergone anti-reflection treatment, 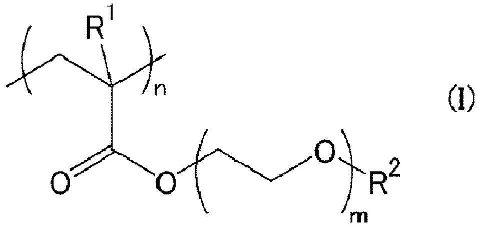 wherein R¹ represents a hydrogen atom or a methyl group; R² represents an alkyl group; m represents 1 to 5; and n represents the number of repetitions.

Embodiment 2. The method of producing a polymer-coated substrate for capturing cancer cells according to Embodiment 1,
wherein the anti-reflection treatment is at least one selected from the group consisting of non-reflective treatment, low reflection treatment, polarizing treatment, and non-glare treatment.

Embodiment 3. The method of producing a polymer-coated substrate for capturing cancer cells according to Embodiment 1 or 2,
wherein the substrate having undergone anti-reflection treatment has a 60-degree gloss of 150% to 50%.

Embodiment 4. The method of producing a polymer-coated substrate for capturing cancer cells according to Embodiment 1 or 2,
wherein the substrate having undergone anti-reflection treatment has a 60-degree gloss of 130% to 70%.

Embodiment 5. The method of producing a polymer-coated substrate for capturing cancer cells according to any combination with any one of Embodiments 1 to 4,
wherein the substrate having undergone anti-reflection treatment is a glass substrate.

Embodiment 6. The method of producing a polymer-coated substrate for capturing cancer cells according to any combination with any one of Embodiments 1 to 5,
wherein the polymer layer has a total thickness of 10 to 1000 nm.

Embodiment 7. The method of producing a polymer-coated substrate for capturing cancer cells according to any combination with any one of Embodiments 1 to 6,
wherein the forming a polymer layer includes forming two or more layers of the polymer layer using a hydrophilic polymer represented by the formula (I) on the surface of the substrate having undergone anti-reflection treatment.

Embodiment 8. The method of producing a polymer-coated substrate for capturing cancer cells according to Embodiment 7,
wherein the two or more polymer layers are formed using the same hydrophilic polymer, and
a number average molecular weight of the hydrophilic polymer constituting a polymer layer on the surface of the substrate having undergone anti-reflection treatment among the two or more polymer layers is larger than a number average molecular weight of the hydrophilic polymer constituting any other polymer layer among the two or more polymer layers.

Embodiment 9. The method of producing a polymer-coated substrate for capturing cancer cells according to Embodiment 8,
wherein the number average molecular weight of the hydrophilic polymer constituting any other polymer layer is 30000 or less.

## Claims

1. A method of producing a polymer-coated substrate for capturing cancer cells, the method comprising
forming a polymer layer using a hydrophilic polymer represented by the following formula (I) on a surface of a substrate having undergone anti-reflection treatment, 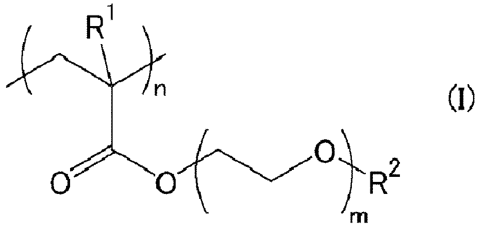 wherein R¹ represents a hydrogen atom or a methyl group; R² represents an alkyl group; m represents 1 to 5; and n represents the number of repetitions.

2. The method of producing a polymer-coated substrate for capturing cancer cells according to claim 1,
wherein the anti-reflection treatment is at least one selected from the group consisting of non-reflective treatment, low reflection treatment, polarizing treatment, and non-glare treatment.

3. The method of producing a polymer-coated substrate for capturing cancer cells according to claim 1 or 2,
wherein the substrate having undergone anti-reflection treatment has a 60-degree gloss of 150% to 50%.

4. The method of producing a polymer-coated substrate for capturing cancer cells according to claim 1 or 2,
wherein the substrate having undergone anti-reflection treatment has a 60-degree gloss of 130% to 70%.

5. The method of producing a polymer-coated substrate for capturing cancer cells according to any one of claims 1 to 4,
wherein the substrate having undergone anti-reflection treatment is a glass substrate.

6. The method of producing a polymer-coated substrate for capturing cancer cells according to any one of claims 1 to 5,
wherein the polymer layer has a total thickness of 10 to 1000 nm.

7. The method of producing a polymer-coated substrate for capturing cancer cells according to any one of claims 1 to 6,
wherein the forming a polymer layer includes forming two or more layers of the polymer layer using a hydrophilic polymer represented by the formula (I) on the surface of the substrate having undergone anti-reflection treatment.

8. The method of producing a polymer-coated substrate for capturing cancer cells according to claim 7,
wherein the two or more polymer layers are formed using the same hydrophilic polymer, and
a number average molecular weight of the hydrophilic polymer constituting a polymer layer on the surface of the substrate having undergone anti-reflection treatment among the two or more polymer layers is larger than a number average molecular weight of the hydrophilic polymer constituting any other polymer layer among the two or more polymer layers.

9. The method of producing a polymer-coated substrate for capturing cancer cells according to claim 8,
wherein the number average molecular weight of the hydrophilic polymer constituting any other polymer layer is 30000 or less.
